# EUROPEAN PATENT SPECIFICATION

(11) **EP 3 066 979 B1**
(45) Date of publication and mention of the grant of the patent: **01.11.2017**
(21) Application number: 16158001.4
(22) Date of filing: 24.05.2011
(51) Int. Cl.: G01N 33/68

(54) **METHODS OF DETERMINING AMYLOID BETA TURNOVER IN BLOOD**
VERFAHREN ZUR BESTIMMUNG DES AMYLOID-BETA-UMSATZES IM BLUT
PROCÉDÉS DE DÉTERMINATION DE RENOUVELLEMENT D'AMYLOÏDE BETA DANS LE SANG

(30) Priority: 24.05.2010 US 347554 P
(43) Date of publication of application: 14.09.2016
(62) Divisional of application: 11787251.5
(73) Proprietor: The Washington University, St. Louis, MO 63130 (US)
(72) Inventor: Bateman, Randall J., St. Louis, MO 63130 (US); Holtzman, David M., St. Louis, MO 63130 (US); Mawuenyega, Kwasi G., St. Louis, MO 63130 (US)
(74) Representative: Spinner, David Richard

(56) References cited:
- WO-A1-2006/017812
- WO-A2-03/068919
- RANDALL J BATEMAN ET AL: "Human amyloid-[beta] synthesis and clearance rates as measured in cerebrospinal fluid in vivo", NATURE MEDICINE, vol. 12, no. 7, 25 June 2006 (2006-06-25), pages 856-861, XP055085288, ISSN: 1078-8956, DOI: 10.1038/nm1438

### SEQUENCE LISTING

<110> THE WASHINGTON UNIVERSITY
   BATEMAN, Randall J.
   HOLTZMAN, David M.
   MAWUENYEGA, Kwasi G.
<120> METHODS OF DETERMINING AMYLOID BETA TURNOVER IN BLOOD
<130> DS/P2211EP01
<140> PCT/US2011/037754
   <141> 2011-05-24
<150> US 61/347,554
   <151> 2010-05-24
<160> 7
<170> PatentIn version 3.5
<210> 1
   <211> 42
   <212> PRT
   <213> Homo sapiens
<400> 1
<210> 2
   <211> 40
   <212> PRT
   <213> Homo sapiens
<400> 2
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val
           35                  40
<210> 3
   <211> 5
   <212> PRT
   <213> Homo sapiens
<400> 3
   Asp Ala Glu Phe Arg
   1               5
<210> 4
   <211> 11
   <212> PRT
   <213> Homo sapiens
<400> 4
   His Asp Ser Gly Tyr Glu Val His His Gln Lys
   1               5                   10
<210> 5
   <211> 12
   <212> PRT
   <213> Homo sapiens
<400> 5
   Leu Val Phe Phe Ala Glu Asp Val Gly Ser Asn Lys
   1               5                   10
<210> 6
   <211> 14
   <212> PRT
   <213> Homo sapiens
<400> 6
   Gly Ala Ile Ile Gly Leu Met Val Gly Gly Val Val Ile Ala
   1               5                   10
<210> 7
   <211> 12
   <212> PRT
   <213> Homo sapiens
<400> 7
   Gly Ala Ile Ile Gly Leu Met Val Gly Gly Val Val
   1               5                   10

## Description

### FIELD OF THE INVENTION

The invention encompasses a method for determining Aβ metabolism in blood.

### BACKGROUND OF THE INVENTION

Alzheimer disease (AD) is the most common cause of dementia and is an increasing public health problem. It is currently estimated to afflict 5 million people in the United States, with an expected increase to 13 million by the year 2050. AD leads to loss of memory, cognitive function, and ultimately independence. AD takes a heavy personal and financial toll on the patient and the family. Because of the severity and increasing prevalence of the disease in the population, it is urgent that better treatments be developed and delivered at the earliest stage of the disease.

### BRIEF DESCRIPTION OF THE FIGURES

**FIG. 1** depicts a graph showing the percent labeled total Aβ in blood over 36 to 48 hours for 6 participants (blue circles) and percent labeled total Aβ in CSF in twelve participants (red squares). Note the rapid rise to plateau by 9 hours with a rapid clearance rate in blood, while CSF does not approach plateau until 18 hours or later. Also note the much more rapid clearance of blood Aβ compared to CSF Aβ. There also may be a second peak of labeled Aβ in blood from 20 to 30 hours (peak ∼26 hours).

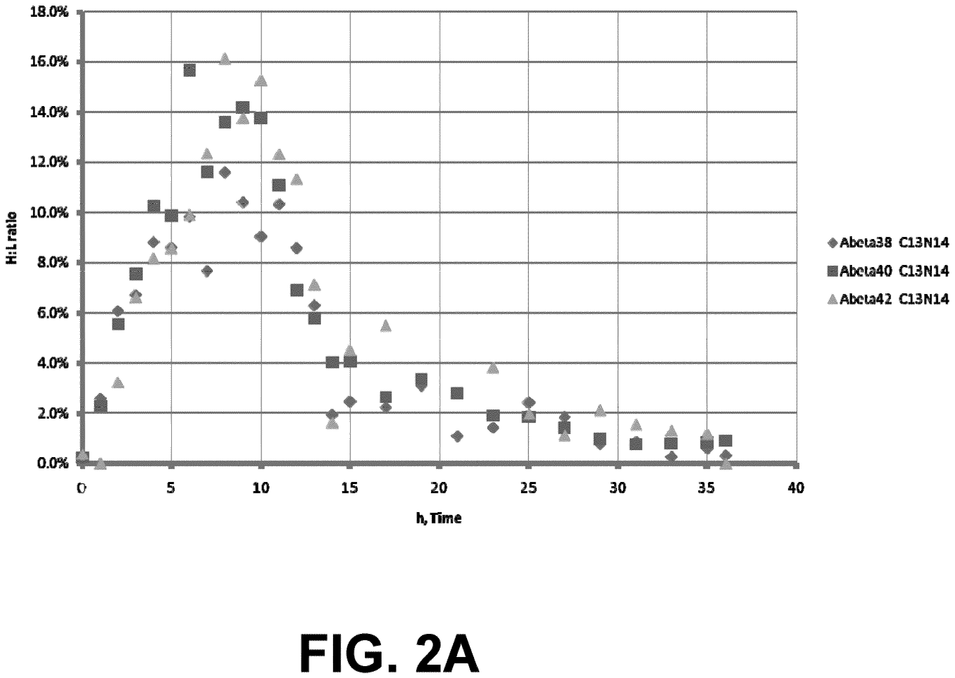

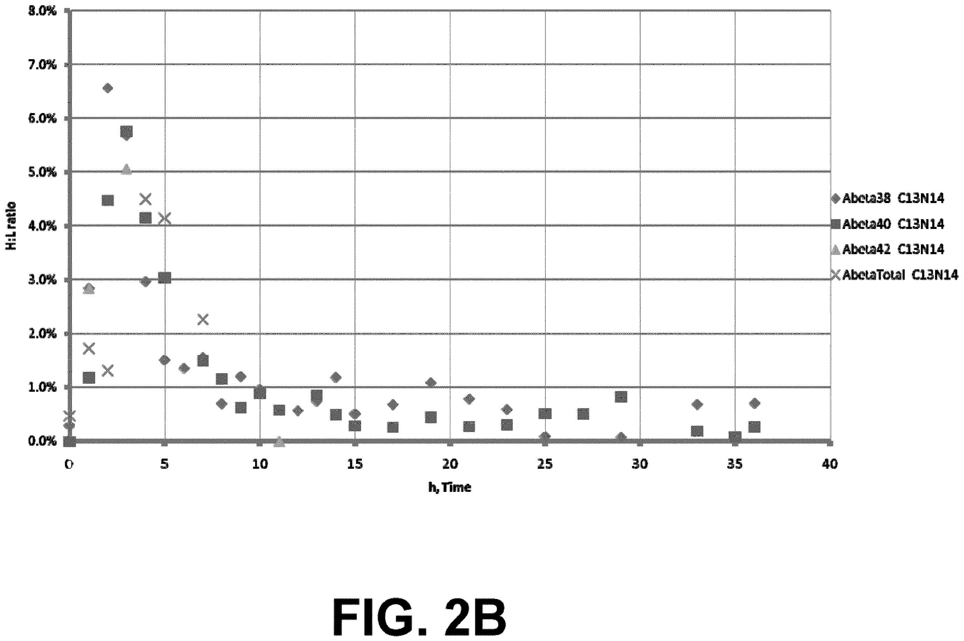

**FIG. 2** depicts percent labeled Aβ in blood over 40 hours after (**a**) intravenous labeling and (**b**) oral labeling.
**FIG. 3** depicts absolute quantitation of blood Aβ isoforms over 40 hours. Labeled N¹⁵ Amyloid-beta internal standards (ISTD) were added to the sample before processing. Samples were processed as described in the examples, and absolute amounts of amyloid beta isoforms are plotted.

### DETAILED DESCRIPTION OF THE INVENTION

The present invention provides a method for determining Aβ metabolism by measuring Aβ turnover in a blood sample. As used herein, "turnover" refers in one embodiment to the rate of labeled Aβ production, the rate of labeled Aβ clearance, or a combination of both, as detailed below. In each instance, however, determining Aβ metabolism requires a measurement of labeled Aβ in a sample. In another embodiment, "turnover" refers to the half-life of Aβ in blood. As used herein, "Aβ" refers to total amyloidβ, an amyloidβ isoform such as Aβ₃₈, Aβ₄₀, or Aβ₄₂, or a combination thereof. Advantageously, a method of the invention eliminates the need for a more difficult to obtain and invasive central nervous system sample to determine the metabolism of Aβ.

### I. Method for determining Aβ metabolism in blood

One aspect of the present invention is a method for determining Aβ metabolism in blood. Importantly, the turnover of Aβ in blood may reflect the metabolism of Aβ in the central nervous system. (This is contrary to steady-state blood Aβ levels, which have not been successfully correlated to central nervous system Aβ.) Generally speaking, measuring the turnover of Aβ in blood comprises administering a label to a subject, collecting a blood sample from the subject, and analyzing the sample to determine the turnover of Aβ in the sample. Each step is described in more detail below.

### (a) administering a label

A method of the invention comprises, in part, administering a label to a subject. Suitable labels and suitable methods of administration are detailed below.

### i. labels

Suitable labels should allow the measurement of the turnover of Aβ in the blood. By way of non-limiting example, a suitable label may include a labeled amino acid or a labeled amino acid precursor. In one embodiment, the label may comprise at least one labeled amino acid. The amino acid may be naturally occurring, synthetic, or an amino acid analogue. In each instance, however, the amino acid should be capable of incorporation into Aβ to allow detection and quantification of the turnover of Aβ in blood. Non-limiting examples of natural amino acids may include alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine. In one embodiment, the amino acid may be an essential amino acid (e.g., an amino acid not produced by the body). In another embodiment, the amino acid may be a non-essential amino acid. In yet another embodiment, a label may comprise at least one essential amino acid and at least one non-essential amino acid. Generally, the choice of amino acid may be based on a variety of factors, including (1) whether the amino acid is present in at least one residue of the protein or peptide of interest; (2) whether the amino acid can quickly reach the site of protein synthesis; (3) whether the labeled amino acid affects the metabolism of the protein of interest (e.g., very large doses of leucine may affect muscle metabolism); and (4) the availability of the desired amino acid may be considered (i.e., some amino acids are much more expensive or harder to manufacture than others).

In some embodiments, a label may comprise at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, or more than twenty different kinds of labeled amino acids.

Amino acids may be labeled using any method known in the art, as long as the amino acid may be incorporated into Aβ and allows the detection of the turnover of Aβ in the blood. Labels may be radioactive or non-radioactive. For instance, in one embodiment, an amino acid may be labeled with a stable non-radioactive isotope. Non-limiting examples may include ²H, ¹³C, ¹⁵N, ¹⁷O, ¹⁸O, ³³S, ³⁴S or ³⁶S. Additionally, it is recognized that a number of other stable isotopes that change the mass of an atom by more or less neutrons than is seen in the prevalent native form would also be effective. In another embodiment, an amino acid may be labeled with more than one label (for instance, ¹³C and ¹⁵N).

In one embodiment, ¹³C₆-phenylalanine, which contains six ¹³C atoms, may be used to label Aβ. In an exemplary embodiment, ¹³C₆-leucine may be used to label Aβ. In yet another embodiment, a combination of a stable non-radioactive isotope and an amino acid listed in **Table A** may be used to label Aβ.

There are numerous commercial sources of labeled amino acids. Generally, the labeled amino acids may be produced either biologically or synthetically. Biologically produced amino acids may be obtained from an organism (e.g., kelp/seaweed) grown in an enriched mixture of ¹³C, ¹⁵N, or other isotope that is incorporated into amino acids as the organism produces proteins. The amino acids are then separated and purified. Alternatively, amino acids may be made with known synthetic chemical processes.

**Table A**

| | |
|---|---|
| ²H | alanine |
| ¹³C | alanine |
| ¹⁵N | alanine |
| ¹⁷O and/or ¹⁸O | alanine |
| ³³S and/or ³⁴S and/or ³⁶S | alanine |
| ²H | arginine |
| ¹³C | arginine |
| ¹⁵N | arginine |
| ¹⁷O and/or ¹⁸O | arginine |
| ³³S and/or ³⁴S and/or ³⁶S | arginine |
| ²H | asparagine |
| ¹³C | asparagine |
| ¹⁵N | asparagine |
| ¹⁷O and/or ¹⁸O | asparagine |
| ³³S and/or ³⁴S and/or ³⁶S | asparagine |
| ²H | aspartic acid |
| ¹³C | aspartic acid |
| ¹⁵N | aspartic acid |
| ¹⁷O and/or ¹⁸O | aspartic acid |
| ³³S and/or ³⁴S and/or ³⁶S | aspartic acid |
| ²H | cysteine |
| ¹³C | cysteine |
| ¹⁵N | cysteine |
| ¹⁷O and/or ¹⁸O | cysteine |
| ³³S and/or ³⁴S and/or ³⁶S | cysteine |
| ²H | glutamic acid |
| ¹³C | glutamic acid |
| ¹⁵N | glutamic acid |
| ¹⁷O and/or ¹⁸O | glutamic acid |
| ³³S and/or ³⁴S and/or ³⁶S | glutamic acid |
| ²H | glutamine |
| ¹³C | glutamine |
| ¹⁵N | glutamine |
| ¹⁷O and/or ¹⁸O | glutamine |
| ³³S and/or ³⁴S and/or ³⁶S | glutamine |
| ²H | glycine |
| ¹³C | glycine |
| ¹⁵N | glycine |
| ¹⁷O and/or ¹⁸O | glycine |
| ³³S and/or ³⁴S and/or ³⁶S | glycine |
| ²H | histidine |
| ¹³C | histidine |
| ¹⁵N | histidine |
| ¹⁷O and/or ¹⁸O | histidine |
| ³³S and/or ³⁴S and/or ³⁶S | histidine |
| ²H | isoleucine |
| ¹³C | isoleucine |
| ¹⁵N | isoleucine |
| ¹⁷O and/or ¹⁸O | isoleucine |
| ³³S and/or ³⁴S and/or ³⁶S | isoleucine |
| ²H | leucine |
| ¹³C | leucine |
| ¹⁵N | leucine |
| ¹⁷O and/or ¹⁸O | leucine |
| ³³S and/or ³⁴S and/or ³⁶S | leucine |
| ²H | lysine |
| ¹³C | lysine |
| ¹⁵N | lysine |
| ¹⁷O and/or ¹⁸O | lysine |
| ³³S and/or ³⁴S and/or ³⁶S | lysine |
| ²H | methionine |
| ¹³C | methionine |
| ¹⁵N | methionine |
| ¹⁷O and/or ¹⁸O | methionine |
| ³³S and/or ³⁴S and/or ³⁶S | methionine |
| ²H | phenylalanine |
| ¹³C | phenylalanine |
| ¹⁵N | phenylalanine |
| ¹⁷O and/or ¹⁸O | phenylalanine |
| ³³S and/or ³⁴S and/or ³⁶S | phenylalanine |
| ²H | proline |
| ¹³C | proline |
| ¹⁵N | proline |
| ¹⁷O and/or ¹⁸O | proline |
| ³³S and/or ³⁴S and/or ³⁶S | proline |
| ²H | serine |
| ¹³C | serine |
| ¹⁵N | serine |
| ¹⁷O and/or ¹⁸O | serine |
| ³³S and/or ³⁴S and/or ³⁶S | serine |
| ²H | threonine |
| ¹³C | threonine |
| ¹⁵N | threonine |
| ¹⁷O and/or ¹⁸O | threonine |
| ³³S and/or ³⁴S and/or ³⁶S | threonine |
| ²H | tryptophan |
| ¹³C | tryptophan |
| ¹⁵N | tryptophan |
| ¹⁷O and/or ¹⁸O | tryptophan |
| ³³S and/or ³⁴S and/or ³⁶S | tryptophan |
| ²H | tyrosine |
| ¹³C | tyrosine |
| ¹⁵N | tyrosine |
| ¹⁷O and/or ¹⁸O | tyrosine |
| ³³S and/or ³⁴S and/or ³⁶S | tyrosine |
| ²H | valine |
| ¹³C | valine |
| ¹⁵N | valine |
| ¹⁷O and/or ¹⁸O | valine |
| ³³S and/or ³⁴S and/or ³⁶S | valine |

### ii. methods of administration

Suitable methods of administering a label may include any method that allows the measurement of the turnover of Aβ in blood. In one embodiment, a label may be administered parenterally, e.g. intravenously, intra-arterially, subcutaneously, intraperitoneally, or intramuscularly. In yet another embodiment, a label may be administered orally.

A label may be administered in several different ways, depending in part, on the type of label. In one embodiment, a label may be administered as an infusion over time. For instance, a label may be administered via an infusion for about 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 minutes. Alternatively, a label may be administered via an infusion for about 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.25, 10.5, 10.75, 11, 11.25, 11.5, 11.75, 12, 12.25, 12.5, or 12.75 hours. In still other embodiments, a label may be administered as an infusion for more than 13 hours. In an exemplary embodiment, the label may be administered as an IV infusion.

A label may also be administered in one or more boluses. For instance, a label may be administered in one bolus, two boluses, three boluses, four boluses, five boluses, or more than five boluses. Generally speaking, a bolus is a discrete administration for a duration of no longer than about 1 hour. In one embodiment, a bolus is administered in 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 seconds. In another embodiment, a bolus is administered in 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 minutes. The time between boluses may be seconds, minutes, hours, or days. For instance, the time between boluses may be about 1, 2, 3, 4, 5, 6, 7, .8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 sec. Alternatively, the time between boluses may be about 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 minutes. In another alternative, the time between boluses may be about 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.25, 10.5, 10.75, 11, 11.25, 11.5, 11.75, 12, 12.25, 12.5, 12.75, 13, 13.25, 13.5, 13.75, 14, 14.25, 14.5,14.75, 15, 15.25, 15.5, 15.75, 16, 16.25, 16.5, 16.75, 17, 17.25, 17.5, 17.75, 18, 18.25, 18.5, 18.75, 19, 19.25, 19.5, 19.75, 20, 20.25, 20.5, 20.75, 21, 21.25, 21.5, 21.75, 22, 22.25, 22.5, 22.75, 23, 23.25, 23.5, 23.75, 24, 24.25, 24.5, 24.75, 25, 25.25, 25.5, 25.75, 26, 26.25, 26.5, 26.75, 27, 27.25, 27.5, 27.75, 28, 28.25, 28.5, 28.75, 29, 29.25, 29.5, 29.75, 30, or more than 30 hours.

In an exemplary embodiment, the label may be administered in at least one oral or IV bolus. In certain embodiments, a label may be administered via a combination of a bolus and an infusion. For instance, a subject may be administered a bolus of label, followed by an infusion of label.

Those of skill in the art will appreciate that the amount (or dose) of the label can and will vary, depending in part on the route of administration and the label used. Generally speaking, the amount should be sufficient to allow the measurement of Aβ turnover in blood. In some embodiments, if the label is administered via infusion, the amount may be between about 0.01 mg/kg/hr to about 4.5 mg/kg/hr. For instance, the amount may be about 0.001, 0.005, 0.01, 0.15, 0.2, 0.25, 0.5, 0.75, 1, 1.25, 1.5, 1.75, 2, 2.25, 2.5, 2.75, 3, 3.25, 3.5, 3.75, 4, 4.25, or 4.5 mg/kg/hr. In other embodiments, if the label is administered via bolus, the amount may be between about 0.01 g to about 8 g. For example, the amount may be 0.001, 0.005, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 7 or 8 g.

### (b) collecting a blood sample

A method of the invention comprises, in part, collecting at least one blood sample from a subject. The blood sample typically contains labeled Aβ. The sample may also contain unlabeled Aβ. As used herein, "blood" refers to whole blood, plasma, or serum. Methods of collecting a blood sample are well known in the art. In an exemplary embodiment, venipuncture, with or without a catheter, may be used to collect a blood sample. In another exemplary embodiment, a finger stick, or the equivalent, may be used to collect a blood sample.

Generally speaking a blood sample is collected after the administration of a label, described in section I (a) above. A skilled artisan will appreciate that the time a blood sample is collected (after administration of a label) can and will vary depending on the label and the method of administering the label. In one embodiment, a blood sample is collected between about 1 min and about 48 hours after administration of a label. For instance, a blood sample may be collected between about 1 min and 48 hours, between about 1 min and 10 hours, or between about 1 min and 30 hours. In another embodiment, a blood sample may be collected between about 10 min and about 24 hours after administration of a label. In yet another embodiment, a blood sample may be collected between about 10 min and about 10 hours after administration of a label. For instance, a blood sample may be collected between about 15 min and 8 hours, between about 15 min and 6 hours, between about 15 min and 4 hours, between about 30 min and 5 hours, between about 1 hour and 5 hours, between about 1 hour and 4 hours, between about 2 hours and 4 hours, or between about 2 hours and 6 hours. In still another embodiment, a blood sample may be collected between about 10 hours and about 20 hours after administration of a label. For example, a blood sample may be collected between about 12 hours and 20 hours, between about 14 hours and 20 hours, between about 16 hours and 20 hours, or about 18 hours and 20 hours. In yet still another embodiment, a blood sample may be collected between about 20 hours and about 30 hours after administration of a label. For instance, a blood sample may be collected between about 22 hours and 28 hours or between about 24 hours and 28 hours. In an alternative embodiment, a blood sample may be collected after 30 hours after administration of a label. For instance, a blood sample may be collected after 30, 32, 34, 36, 38, 40, 42, 44, 46, 48 or greater than 48 hours after administration of a label.

In some embodiments, a blood sample may be collected after about 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 sec after administration of a label. In other embodiments, a blood sample may be collected after about 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 minutes after administration of a label. In certain embodiments, a blood sample may be collected after about 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.25, 10.5, 10.75, 11, 11.25, 11.5, 11.75, 12, 12.25, 12.5, 12.75, 13, 13.25, 13.5, 13.75, 14, 14.25, 14.5,14.75, 15, 15.25, 15.5, 15.75, 16, 16.25, 16.5, 16.75, 17, 17.25, 17.5, 17.75, 18, 18.25, 18.5, 18.75, 19, 19.25, 19.5, 19.75, 20, 20.25, 20.5, 20.75, 21, 21.25, 21.5, 21.75, 22, 22.25, 22.5, 22.75, 23, 23.25, 23.5, 23.75, 24, 24.25, 24.5, 24.75, 25, 25.25, 25.5, 25.75, 26, 26.25, 26.5, 26.75, 27, 27.25, 27.5, 27.75, 28, 28.25, 28.5, 28.75, 29, 29.25, 29.5, 29.75, or 30 hours after administration of a label.

In exemplary embodiments, if the label is administered intravenously for about 9 hours, a blood sample may be collect between about one hour and about 15 hours, between about 5 hours and about 13 hours, or between about 8 hours and about ten hours after administration of the label. In one exemplary embodiment, if the label is administered intravenously, a blood sample may be collected about nine hours after administration of the label. In other exemplary embodiments, if the label is administered orally, a blood sample may be collected between about 30 min and about 5 hours or between about 1 hour and four hours after administration of the label. In a certain exemplary embodiment, if the label is administered orally, a blood sample may be collected about three hours after administration of the label.

More than one blood sample may be collected from a subject. For instance, 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, or more than 40 blood samples may be collected from a subject. The time between samples may be seconds, minutes, hours, or days. For instance, the time between samples may be about 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 sec. Alternatively, the time between samples may be about 1, 2, 3, 4, 5, 6, 7, 8, 9, 10, 11, 12, 13, 14, 15, 16, 17, 18, 19, 20, 21, 22, 23, 23, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 minutes. In another alternative, the time between samples may be about 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.25, 10.5, 10.75, 11, 11.25, 11.5, 11.75, 12, 12.25, 12.5, 12.75, 13, 13.25, 13.5, 13.75, 14, 14.25, 14.5,14.75, 15, 15.25, 15.5, 15.75, 16, 16.25, 16.5, 16.75, 17, 17.25, 17.5, 17.75, 18, 18.25, 18.5, 18.75, 19, 19.25, 19.5, 19.75, 20, 20.25, 20.5, 20.75, 21, 21.25, 21.5, 21.75, 22, 22.25, 22.5, 22.75, 23, 23.25, 23.5, 23.75, 24, 24.25, 24.5, 24.75, 25, 25.25, 25.5, 25.75, 26, 26.25, 26.5, 26.75, 27, 27.25, 27.5, 27.75, 28, 28.25, 28.5, 28.75, 29, 29.25, 29.5, 29.75, 30, or more than 30 hours.

The blood sample should typically be large enough to allow the measurement of Aβ turnover. More than one sample may be pooled for a particular time point. In one embodiment, a blood sample may be about 50µL to about 3000µL. For instance, a blood sample may be about 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2050, 2100, 2150, 2200, 2250, 2300, 2350, 2400, 2450, 2500, 2550, 2600, 2650, 2700, 2750, 2800, 2850, 2900, 2950, or 3000 µL. In other embodiments, a blood sample may be more than 3000µL.

### (c) analyzing the sample to determine the turnover of Aβ

A method of the invention, comprises, in part, analyzing the sample to determine the turnover of Aβ in blood. Generally speaking, analyzing the sample encompasses (a) detecting and quantifying labeled and unlabeled Aβ in a blood sample and (b) deriving the turnover of Aβ from the quantity of labeled and unlabeled Aβ in the blood sample. Each step is discussed in more detail below.

### i. detecting and quantifying labeled and unlabeled Aβ in a blood sample

The method of detecting and quantifying labeled and unlabeled Aβ in a blood sample can and will vary. For instance, detection methods will vary with the type of label used (e.g. radioactive or non-radioactive). In one embodiment, if a non-radioactive label is used, the detection method should be sensitive enough to detect changes in mass of the labeled protein with respect to the unlabeled protein. For instance, labeled and unlabeled Aβ may be detected with mass spectrometry. In one embodiment, the mass spectrometry protocol outlined in the Examples is used.

Additional techniques may be used to separate the labeled and unlabeled Aβ from other blood components, or to concentrate the labeled and unlabeled Aβ in a sample. For instance, labeled and unlabeled Aβ may be immunoprecipitated from a blood sample. Protocols for immunoprecipitations are known in the art. In one embodiment, the immunoprecipitation antibody may be attached to a solid support, such as a bead or resin. In another embodiment, the immunoprecipitation protocol detailed in the Examples may be used. Other methods of separating or concentrating Aβ may include chromatography. In particular, techniques linking a chromatographic step with a mass spectrometry step may be used.

To aid in detection and quantification of labeled and unlabeled Aβ, the Aβ may be divided into smaller peptides. For instance, Aβ may be digested with a protease to create several small peptides. In one embodiment, trypsin is used to digest Aβ.

Generally speaking, the method of detecting labeled and unlabeled Aβ may also be used to quantify the amount of labeled and unlabeled Aβ. In some embodiments, quantification encompasses determining the ratio between labeled and unlabeled Aβ.

### ii. deriving the turnover of Aβ

From the detection and quantification step outlined above, the turnover of Aβ may be derived. As used herein, "turnover" refers in one embodiment to the rate of labeled Aβ production, the rate of labeled Aβ clearance, or a combination of both. In another embodiment, "turnover" refers to the half-life of Aβ in blood. In still another embodiment, "turnover" refers to the product of the percent labeled Aβ and the absolute quantitation value of Aβ. For instance, "turnover" may refer to the product of the percent labeled Aβ₄₂ to the absolute quantitation value of Aβ₄₂.

Turnover may be calculated, in part, from the change over time in labeled, unlabeled or ratio of labeled to unlabeled Aβ. In other embodiments, turnover may be determined from the peak of labeled Aβ. In still other embodiments, fractional synthesis rate, fractional clearance rate, absolute synthesis rate, absolute clearance rate, half-life, decay rate, or compartmental modeling may be used to determine turnover. In each embodiment, turnover may be calculated using the in vivo labeled Aβ, in vivo unlabled Aβ, the ex vivo internal standard (absolute quantitation), or a combination thereof.

### II. Kit

An alternative embodiment of the invention encompasses a kit. The kit typically comprises a solution of at least one labeled amino acid. In one embodiment, the solution is a shake or drink that may be consumed orally. The kit also contains instructions for administering the labeled amino acid solution and subsequently collecting at least one blood sample. The kit may also contain a container and/or paraphernalia for collecting and storing the blood sample. Additionally, the kit may contain an internal standard to ensure that that blood sample is of adequate quality to be analyzed for Aβ turnover and for absolute quantitation of labeled and unlabeled Aβ.

Yet another aspect of the present application encompasses a business method. Generally speaking, the method comprises distributing a kit of the invention to either a physician or individual. After the kit is used to collect a sample from the subject following the directions included in the kit, the sample is then returned to a lab for processing using a method of the invention.

The following examples are included to demonstrate preferred embodiments of the invention. It should be appreciated by those of skill in the art that the techniques disclosed in the examples that follow represent techniques discovered by the inventors to function well in the practice of the invention. Those of skill in the art should, however, in light of the present disclosure, appreciate that many changes can be made in the specific embodiments that are disclosed and still obtain a like or similar result without departing from the spirit and scope of the invention, therefore all matter set forth or shown in the accompanying drawings is to be interpreted as illustrative and not in a limiting sense.

### DEFINITIONS

As used herein, "bolus" refers to a discrete dosing of label. More than one bolus may be used to administer a label, but each bolus is a discrete dose. This is in contrast to an infusion, which is a continuous dose administered over time.

As used herein, "central nervous system" refers to the brain, cerebral spinal fluid, or any other tissue or fluid of the central nervous system where Aβ may be found.

As used herein, "infusion" refers to a continuous dosing of label over time. In some embodiments, the rate of the dosing may change over time.

As used herein, "metabolism" refers to any combination of the synthesis, transport, breakdown, modification, or clearance rate of a biomolecule.

As used herein, "subject" refers to any subject that has a central nervous system. In exemplary embodiments, "subject" refers to any subject that is susceptible to a disease or disorder characterized by amyloid plaques. In one exemplary embodiment, "subject" refers to an individual with AD or an individual that is at risk for AD. In another exemplary embodiment, "subject" refers to an individual that is advanced in age.

### EXAMPLES

The following examples illustrate various iterations of the invention.

A pioneering approach was recently developed to directly measure Aβ metabolism in the central nervous system of living humans (Bateman et. al 2006). This method requires participants be admitted to a research hospital room, and have two IV catheters and a lumbar spinal catheter placed so that hourly samples of blood and cerebral-spinal fluid can be obtained. Using this method, recent studies have demonstrated that Aβ has a rapid metabolism (half-life of 8-10 hours) in the human brain and cerebral-spinal fluid (CSF).

Blood Aβ dynamics, however, are not well understood as previously there was no method to measure labeled Aβ within blood. If such a method were available, the physiology (and pathophysiology) of Aβ could be better understood as a quantitative measure of Aβ production in the brain, transport to blood and cerebral-spinal fluid, and clearance from blood. A blood labeled Aβ assay would allow for the physiology and pathophysiology of Aβ to be measured without invasive spinal catheters.

A stable isotope labeling kinetics immunoprecipitation-mass spectrometry approach was developed to measure labeled blood Aβ. This provides the ability to measure blood Aβ production, transport between compartments, and clearance rates in humans.

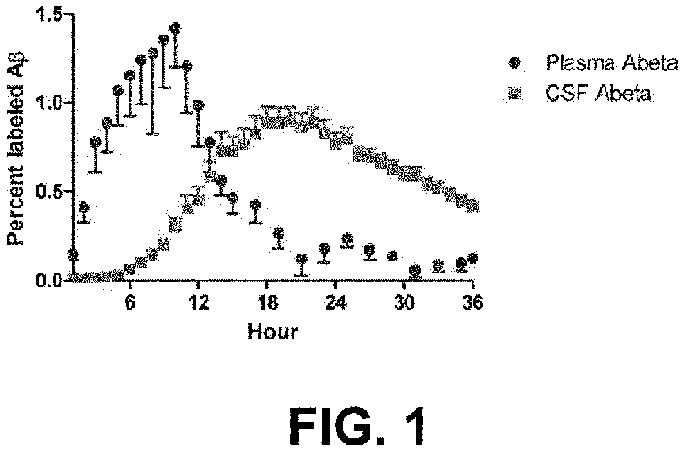

The half-life of blood/plasma Aβ is distinctly different than in the CNS/CSF, with a t1/2 of 1 to 3 hours in production **(****FIG. 1**). This contrasts with a half-life of 8 to 10 hours as measured in CSF (Bateman et. al 2006). For **FIG. 1**, subjects were administered a labeled amino acid (¹³C₆ leucine; infused over 9 hours with a 10 minute primed infusion 2 mg/kg, followed by 2 mg/kg/hour for 8 hours and 50 minutes) and then blood samples were taken every hour for 0 - 15 hr, then every odd hour to hour 35, then at 36 & 48 hours. Twenty-eight samples were taken in total. The samples were stored frozen. Then, samples were thawed and a protease inhibitor cocktail was added. Samples were centrifuged and then pooled from two samples of plasma (2 mls total) at every time point. Next, Aβ was immunoprecipitated from the samples as described below:
Day 1:
   1. Samples thawed on ice.
   2. Add 20 µl of Complete Protease Inhibitors (Roche, 1 tab dissolved in 1 ml water)
   3. Centrifuge at 14,000 RPM at 4°C for 15 minutes to remove particulates.
   4. Pool the supernatants.
   5. Dilute media standards into PBS.
   6. Wash HJ5.1 (Aβ specific antibody) beads twice with 1 x PBS with 0.02% azide. Make 50% bead slurry at the end.
   7. Add 220 µl 5M guanidine hydrochloride solution.
   8. Add 20 ul Tween-20 (5% in PBS for 0.05% final)
   9. Add 5 ul of ISTD (N¹⁵- Aβ).
   10.Add 30 µl antibody- beads (50% slurry enough for ∼20ng Aβ).
   11. Incubate overnight at 4°C.
Day 2:
   12. Centrifuge beads at 4500 RPM for 5 minutes.
   13. Remove supernatant into a new tube and save (plasma only).
   14.Add 1 ml 0.5M guanidine hydrochloride solution
   15. Add 10 ul of 5% for 0.05% final Tween-20.
   16. Once re-suspended transfer beads to 1.5 ml tube.
   17. Centrifuge beads at 4500 RPM for 5 minutes.
   18. Discard supernatant.
   19. Wash the beads two times with 1 x AmBiC rinse. Discard the supernant and dry beads
   20.Add 50 µl (bead volume) 98% formic acid (elution solution) and centrifuge beads. Remove supernatant and place in 1.5 ml tube.
   21. Dry the elution solution in a speedvac for 1 hour to remove formic acid (37°C).
   23. Thaw 20 µg trypsin. Add 1 ml of 25mM AmBiC solution to re-suspend the trypsin to a final concentration of 20 ng/ul.
   24. Re-suspend the proteins in 10 ul solution of 25 mM AmBiC.
   25. Add 10 µl of the 20 ng/µl trypsin solution to the beads for digestion.
   26. Digest O/N (16 hrs) at 37°C in the incubator.
DAY 3:
   27. Perform a short spin of the samples to let condensate settle at the bottom of tube.
   28.Add 2 ul of formic acid to the digest (to precipitate proteins)
   29. Mix samples.
   30. Centrifuge the tryptic digest at 14,000 RPM at 4°C for 15 minutes.
   31.Transfer to autosampler vials then spin down samples using the speedvac and keep in the refrigerator at 4°C.

The samples were then analyzed by mass spectrometry as follows:

The amino acid sequence of Aβ 1-42 is DAEFRHDSGYEVHH QKLVFFAEDVGSNKGAIIGLMVGGVVIA (SEQ ID NO:1) and Aβ 1-40 is DAEFRHDSGYEVH HQKLVFFAEDVGSNKGAIIGLMVGGVV (SEQ ID NO:2). When it is digested with a protease like trypsin, four smaller peptides are generated as Aβ 1-5 DAEFR (SEQ ID NO:3), Aβ 6-16 HDSGYEVHHQK (SEQ ID NO:4), Aβ 17-28 LVFFAEDVGSNK (SEQ ID NO:5), and Aβ 29-42 GAIIGLMVGGVVIA (SEQ ID NO:6). Only two of these peptides are of relevance for quantitation using the SILK technology (Stable Isotope Labeling Kinetics - Bateman et. al 2009) with leucine labeling because their sequence includes a leucine residue that can be labeled in vivo. These peptides are Aβ 17-28 LVFFAEDVGSNK (SEQ ID NO:5), and Aβ 29-42 GAIIGLMVGGVVIA (SEQ ID NO:6), but we use only the Aβ 17-28 LVFFAEDVGSNK (SEQ ID NO:5) peptide because it represents the total amounts of Aβ in the blood and produces a higher signal. Of course, it may be useful to also quantify the c-terminal (Aβ 29-42 GAIIGLMVGGVVIA (SEQ ID NO:6) or Aβ 29-40 GAIIGLMVGGVV (SEQ ID NO:7)) or n-terminal fragments in the blood as well. In general, any amino acid label can be used, any endoprotease can be used (or none at all) to label Aβ & quantify the label in the blood.

Experiments were performed on a TSQ Vantage mass spectrometer coupled to a nanoflow ESI source and a nano-2D liquid chromatography (NanoLC-2D). The samples were maintained at a temperature of 4°C in the autosampler tray. Samples were injected onto a home-made nano column (150-mm diameter) with a pulled tip, packed to 15 cm with Ace 5 C18 AR column packing material (MAC-MOD Analytical, Chadds Ford, PA). Peptides were separated by the Nano2D-LC (Eksigent, Inc. Ultra NanoLC-2D) at a flow rate of 1 mL/ min. Solvent A was 0.1 % formic acid in water and solvent B was 0.1 % formic acid in acetonitrile. The gradient was 15% B to 25% B in 10 min followed by 25% B to 95% B in 5 min, then, ramped down to 15% B in 2 min and the column re-equilibrated for 3 min while the autosampler was picking another sample for injection.

The TSQ Vantage mass spectrometer (MS) was operated in positive ion mode using a spray voltage of 1.2 kV, with optimized parameters from tuning with peptides. Data was acquired in Multiple Reaction Monitoring (MRM) mode. During this MRM experiments, the mass of the peptide was first detected in the first dimension, or as MS1. While MS1 is used to perform quantitation, the technique suffers from lack of specificity especially in very complex matrices like blood and due to the fact that many peptides have the same intact mass. The peptide ions were fragmented and detected in the mass spectrometer and this second dimension of MS fragmentation (MS2) provided unique fragments. The combination of the specific precursor mass and the unique fragment ions were used to selectively monitor the peptide to be quantified. In this case, multiple fragment ions (also known as reactions) were selectively monitored to quantitate Aβ 17-28, resulting in a unique MRM experiment. Aβ 17-28 has a precursor mass (MS1) with a charge to mass ratio of 663.340 for the endogenous peptide and 666.340 for the label incorporated peptide. Three each of their fragment ions were monitored after the MS2 fragmentation. The fragment ions also known as transition ions monitored have a mass to charge ratios of 819.38, 966.45 and 1113.52. The MRM experiments are detected and plotted as single chromatographic peaks which were processed by Xcalibur, which is the instrument control software.

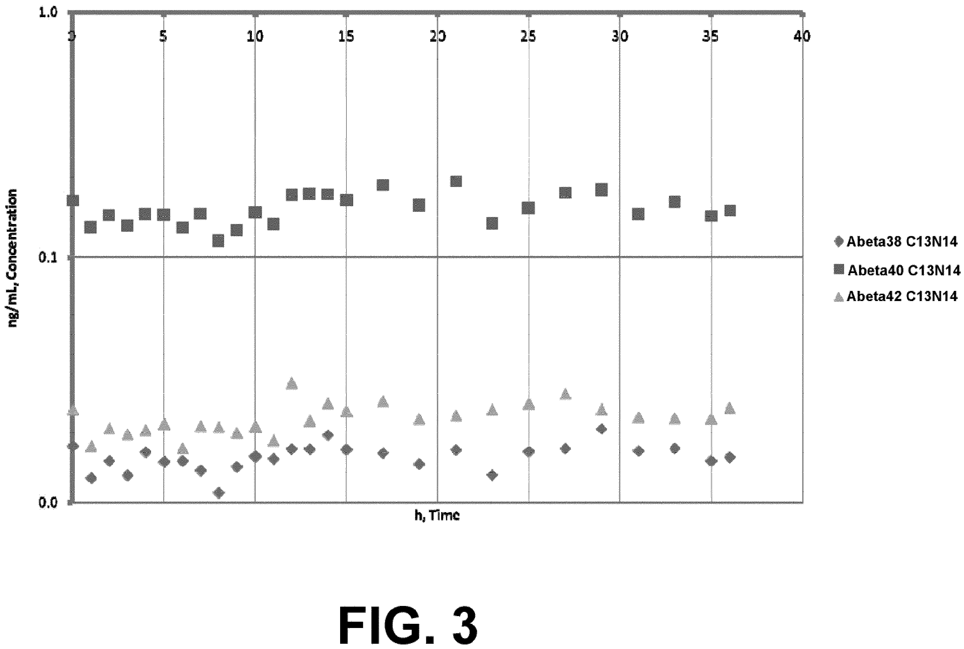

Figure 3 shows the difference between administering an oral (B) label versus an intravenous (A) label. For each of the isoforms tested, the labeling peak occurred between about 1 and 5 hours for oral administration and between about 5 and 10 hours for intravenous administration. Label and samples were administered and collected, respectively, as described above.

Figure 4 shows the absolute quantitation of plasma Aβ isoforms, again measured using the protocol above. Labeled N¹⁵ Amyloid-beta internal standards (ISTD) were added to the sample before processing. Samples were processed as described above.

## Claims

1. An *in vitro* method for measuring the *in vivo* rate of Aβ turnover in the blood of a subject, the method comprising:
(a) determining by mass spectrometry the amount of labelled Aβ, or the amount of labelled Aβ and unlabelled Aβ, in a blood sample obtained from the subject;
(b) calculating the rate of Aβ clearance using the amount of labeled Aβ, or the amount of labeled Aβ and the amount of unlabeled Aβ, determined in step (a), wherein
(i) the rate of Aβ clearance is calculated from the amount of labeled Aβ, or the amount of labeled Aβ and the amount of unlabeled Aβ, in a sample obtained between 10 hours and 20 hours after intravenous administration by a 9 hour infusion has begun of at least one amino acid labelled with a stable non-radioactive isotope to the subject, or
(ii) the rate of Aβ clearance is calculated from the amount of labeled Aβ, or the amount of labeled Aβ and the amount of unlabeled Aβ, in a sample obtained after 4, 5, 6, 7, 8, 9, or 10 hours after oral or intravenous bolus administration of at least one amino acid labelled with a stable non-radioactive isotope to the subject.

2. The method of claim 1, wherein the labelled amino acid was administered orally.

3. The method of claim 2, wherein between about 0.05g and about 8g of amino acid labelled with a stable non-radioactive isotope was administered.

4. The method of claim 1, wherein the labelled amino acid was administered intravenously.

5. The method of claim 4, wherein the labelled amino acid was administered as an infusion at between about 0.01mg/kg/hour and about 3 mg/kg/hour.

6. The method of claim 1, wherein step (a) further comprises initially isolating Aβ from the blood sample.

7. The method of claim 1, wherein the amount of labeled Aβ or unlabeled Aβ is selected from the group consisting of total Aβ, Aβ₃₈, Aβ₄₀, and Aβ₄₂.

8. The method of claim 1, wherein Aβ turnover is calculated from at least two blood samples.

9. The method of claim 1, wherein the stable non-radioactive isotope is selected from the group consisting of ²H, ¹³C, ¹⁵N, ¹⁷O, ¹⁸O, ³³S, ³⁴S, and ³⁶S.

10. The method of claim 9, wherein the stable non-radioactive isotope is ¹³C.

11. The method of claim 1, wherein the labelled amino acid is ¹³C₆-leucine or ¹³C₆-phenylalanine.

## Patentansprüche

1. *In* vitro-Verfahren zum Messen der *in vivo*-Rate eines Aß-Durchsatzes im Blut eines Individuums, wobei das Verfahren umfasst:
(a) Bestimmen der Menge an markiertem Aβ oder der Menge an markiertem Aβ und unmarkiertem Aβ in einer von dem Individuum erhaltenen Blutprobe durch Massenspektrometrie,
(b) Berechnen der Aß-Ausscheidungsrate unter Verwendung der in Schritt (a) bestimmten Menge an markiertem Aβ oder der Menge an markiertem Aβ und der Menge an unmarkiertem Aβ, wobei
(i) die Aß-Ausscheidungsrate berechnet wird aus der Menge an markiertem Aβ oder der Menge an markiertem Aβ und der Menge an unmarkiertem Aβ in einer Blutprobe, die zwischen 10 Stunden und 20 Stunden nach Beginn einer intravenösen Verabreichung mindestens einer mit einem stabilen nicht-radioaktiven Isotop markierten Aminosäure mittels einer 9-Stunden-Infusion an das Individuum erhalten wurde, oder
(ii) die Aß-Ausscheidungsrate berechnet wird aus der Menge an markiertem Aβ oder der Menge an markiertem Aβ und der Menge an unmarkiertem Aβ in einer Blutprobe, die 4, 5, 6, 7, 8, 9, oder 10 Stunden nach oraler oder intravenöser Bolus-Verabreichung mindestens einer mit einem stabilen nicht-radioaktiven Isotop markierten Aminosäure an das Individuum erhalten wurde.

2. Verfahren nach Anspruch 1, wobei die markierte Aminosäure oral verabreicht wurde.

3. Verfahren nach Anspruch 2, wobei zwischen etwa 0,05 g und 8 g mit einem stabilen nicht-radioaktiven Isotop markierter Aminosäure verabreicht wurden.

4. Verfahren nach Anspruch 1, wobei die markierte Aminosäure intravenös verabreicht wurde.

5. Verfahren nach Anspruch 4, wobei die markierte Aminosäure als Infusion mit zwischen etwa 0,01 mg/kg/Stunde und etwa 3 mg/kg/Stunde verabreicht wurde.

6. Verfahren nach Anspruch 1, wobei Schritt (a) ferner das anfängliche Isolieren von Aβ aus der Blutprobe umfasst.

7. Verfahren nach Anspruch 1, wobei die Menge an markiertem Aβ oder unmarkiertem Aβ ausgewählt ist aus der Gruppe, bestehend aus gesamtem Aβ, Aβ₃₈, Aβ₄₀ und Aβ₄₂.

8. Verfahren nach Anspruch 1, wobei der Aß-Durchsatz aus mindestens zwei Blutproben berechnet wird.

9. Verfahren nach Anspruch 1, wobei das stabile nicht-radioaktive Isotop ausgewählt ist aus der Gruppe, bestehend aus ²H, ¹³C, ¹⁵N, ¹⁷O, ¹⁸O, ³³S, ³⁴S, und ³⁶S.

10. Verfahren nach Anspruch 9, wobei es sich bei dem stabilen nicht-radioaktiven Isotop um ¹³C handelt.

11. Verfahren nach Anspruch 1, wobei es sich bei der markierten Aminosäure um ¹³C₆-Leucin oder ¹³C₆-Phenylalanin handelt.

## Revendications

1. Procédé *in vitro* pour mesurer la vitesse *in vivo* de renouvellement de Aβ dans le sang d'un sujet, le procédé comprenant:
(a) la détermination par spectrométrie de masse de la quantité de Aβ marqué, ou de la quantité de Aβ marqué et de Aβ non marqué, dans un échantillon de sang obtenu auprès du sujet;
(b) le calcul de la vitesse de clairance de Aβ en utilisant la quantité de Aβ marqué, ou la quantité de Aβ marqué et la quantité de Aβ non marqué, déterminée dans l'étape (a), où
(i) la vitesse de clairance de Aβ est calculée d'après la quantité de Aβ marqué, ou la quantité de Aβ marqué et la quantité de Aβ non marqué, dans un échantillon obtenu entre 10 heures et 20 heures après que l'administration intraveineuse par une perfusion de 9 heures a commencé d'au moins un acide aminé marqué avec un isotope non radioactif stable au sujet, ou
(ii) la vitesse de clairance de Aβ est calculée d'après la quantité de Aβ marqué, ou la quantité de Aβ marqué et la quantité de Aβ non marqué, dans un échantillon obtenu après 4, 5, 6, 7, 8, 9 ou 10 heures après l'administration de bol orale ou intraveineuse d'au moins un acide aminé marqué avec un isotope non radioactif stable au sujet.

2. Procédé selon la revendication 1, où l'acide aminé marqué a été administré oralement.

3. Procédé selon la revendication 2, où entre environ 0,05 g et environ 8 g d'acide aminé marqué avec un isotope non radioactif stable ont été administrés.

4. Procédé selon la revendication 1, où l'acide aminé marqué a été administré par voie intraveineuse.

5. Procédé selon la revendication 4, où l'acide aminé marqué a été administré sous forme d'une perfusion à raison d'entre environ 0,01 mg/kg/heure et environ 3 mg/kg/heure.

6. Procédé selon la revendication 1, où l'étape (a) comprend en outre initialement l'isolement de Aβ à partir de l'échantillon de sang.

7. Procédé selon la revendication 1, où la quantité de Aβ marqué ou de Aβ non marqué est choisie dans le groupe consistant en Aβ total, Aβ₃₈, Aβ₄₀ et Aβ₄₂.

8. Procédé selon la revendication 1, où le renouvellement de Aβ est calculé à partir d'au moins deux échantillons de sang.

9. Procédé selon la revendication 1, où l'isotope non radioactif stable est choisi dans le groupe consistant en ²H, ¹³C, ¹⁵N, ¹⁷O, ¹⁸O, ³³S, ³⁴S et ³⁶S.

10. Procédé selon la revendication 9, où l'isotope non radioactif stable est ¹³C.

11. Procédé selon la revendication 1, où l'acide aminé marqué est la ¹³C₆-leucine ou la ¹³C₆-phénylalanine.
